(19) **Europäisches Patentamt**
**European Patent Office**
**Office européen des brevets**

(11) **EP 1 859 816 B1**

(12) **EUROPEAN PATENT SPECIFICATION**

(45) Date of publication and mention
of the grant of the patent:
**01.09.2010 Bulletin 2010/35**

(51) Int Cl.:
***A61L 15/28*** (2006.01)    ***A61F 13/02*** (2006.01)

(21) Application number: **05822619.2**

(22) Date of filing: **22.12.2005**

(86) International application number:
**PCT/CN2005/002290**

(87) International publication number:
**WO 2006/097024 (21.09.2006 Gazette 2006/38)**

(54) **THE PREPARING METHOD AND THE USE OF ANTISEPTIC MEDICAL DRESSING**

VERFAHREN ZUR HERSTELLUNG UND VERWENDUNG EINES ANTISEPTISCHEN
MEDIZINISCHEN VERBANDS

PROCEDE DE PREPARATION ET UTILISATION DE PANSEMENT MEDICAL ANTISEPTIQUE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **17.03.2005 CN 200510024442
03.08.2005 CN 200510088100**

(43) Date of publication of application:
**28.11.2007 Bulletin 2007/48**

(73) Proprietor: **Origien Medical Technologies
Zhangjiang Hi-Tech Park
Shanghai 201023 (CN)**

(72) Inventors:
• **LI, Yibin
Shanghai 200127 (CN)**

• **WU, Qingji
Shanghai 200127 (CN)**
• **CHENG, Liping
Shanghai 200127 (CN)**

(74) Representative: **Epping - Hermann - Fischer
Patentanwaltsgesellschaft mbH
Ridlerstrasse 55
80339 München (DE)**

(56) References cited:
**WO-A-01/24840      WO-A-93/12275
CN-A- 1 298 972    CN-A- 1 431 229
CN-A- 1 493 364    US-A- 4 946 870**

**Description**

Field of the Invention

The present invention relates to a method for the preparation of a wound dressing, in particular to a method for the preparation of an anti-microbial wound dressing, and also to the use of said anti-microbial wound dressing.

Background Art

For a long time, wound dressing for surgery is sterile wadding and cotton-based gauze, the use of which suffers from certain limitations. Cotton gauze doesn't possess any anti-microbial characteristics. Although applied as sterile, cotton gauzes can be infected by microbes in the course of using. These traditional dressings tend to adhere to wound and even be integrated into new-born flesh, causing pain and new wound to patient during dressing change. The remaining fragments of these traditional dressings on the wound bed after dressing change are able to affect the healing of wound. Other dressings made of synthetic materials also have same drawbacks as discussed above.

When treating chronic wounds, a wound dressing is desirable to absorb a high volume of exudate and at the same time promote the healing of the wound. The currently widely used alginate fiber dressing doesn't have enough absorption ability to treat wound exuding high volume of exudate.

When treating burn patient, in order to control water evaporation and germ intrusion, typical therapy is covering the wound with dressing after excising dead tissue. Skin from pig and human is proved to be effective burn wound dressing. However, such skin bears the drawback of high cost and rejection to heterogeneous skin.

WO94/16746 discloses a wound dressing comprising carboxymethyl cellulose which is capable of absorbing 15 times its own weight of saline. Such cellulose can be applied during surgery and treating chronic wound. WO99/02093 discloses a wound dressing made of carboxymethyl cellulose and the production method thereof. However, the above two inventions facilitate wound healing only by providing a moist environment. Therefore, it is very meaningful to create an antiphlogistic, hemostatic biological wound dressing for surgery and burn therapy. Such dressing is not adhesive to wound and is absorbable to human tissue, thus could relieve pain of patient.

Polyacetylglucosamine, also known as chitosan, is an amylose widely existing in nature. It is a major composition of fungal cell wall and carapace of shrimps, crabs and insects. It has an unique property of being absorbed by human tissue after hydrolysis by lysozyme. This material is non-toxic, odorless and compatible with human tissue and does not cause any immune response. It also has antibiotic, antiphlogistic, hemostatic and antalgic capability. It facilitates wound healing as well.

Carboxymethyl chitosan is a category of Chitosan derivatives after the carboxylmethylation of chitosan. Among numerous Chitosan derivatives, carboxymethyl chitosan is very important and widely used in food conservatives, cosmetics and pharmaceuticals. In Studies on Carboxymethyl Chitosan's Structure and Anti-microbial Capabilities, L Chen et al. Journal of Wuhan University, April, 2000, the production method of carboxymethyl chitosan and its excellent antimicrobial property is discussed. Chinese invention patent application No. 92106598.7, 03153650.6, 20040015093.1 also disclose the application of carboxymethyl chitosan in different fields.

In addition, US 20050058694 mentions a wound dressing partially composed of carboxymethyl chitosan. The carboxylmethylation is achieved by one-step method of treating chitosan fibers with a water/alcohol solution containing chloroacetic acid and sodium hydroxide. The dressing obtained becomes a semi-transparent gel material in the existence of water and its fiber structure is visible. The high aqueous absorbency of carboxymethyl fiber causes difficulty in fiber opening and web formation during consequent processing of non-woven fabric when carboxymethyl fiber is made into non-woven fabric by non-woven technique. In addition, after being processed by non-woven machine, the fiber finish on the surface of carboxymethyl fiber makes non-woven fabric hydrophobic, which affects the fluid absorbency of dressing.

Disclosure of the Invention

The first technical problem that the present invention solves is to provide a method of preparation of an anti-microbial wound dressing. Said dressing can be applied in surgical wound, burn, and other chronic wound. Said dressing, when covering wound, is able to prevent water in body fluids from losing, provide a favorable moist environment for wound healing and maintain a fluid-free, maceration-free, germ-free wound surface. Said dressing is antiphlogistic, hemostatic and antalgic and promote wound healing.

Another technical problem that the present invention solves is to avoid the disadvantage of hydrophobicity of non-woven fabric made from carboxymethyl fiber by non-woven technique in prior art. The hydrophobicity affects fluid absorbency of the dressing.

The third technical problem that the present invention solves is to overcome the difficulty in fiber opening and web formation during consequent processing of non-woven fabric when carboxymethyl fiber is made into non-woven fabric by non-woven technique, caused by the high aqueous absorbency of carboxymethyl fiber in prior art.

In order to solve the above technical problems, according to the present invention, there is provided a two-step method to produce carboxymethyl chitosan dressing, which comprises:

Contact chitosan fiber with 40-50% NaOH solution with bath ratio being 1:20-60 for 0.5-6 hours at room temperature to obtain alkalized chitosan fiber, wash said alkalized product with absolute ethanol;

Contact said product with chloroacetic acid in isopropanol. The concentration of chloroacetic acid is 20-40%. Reaction temperature is 35-75°C. Reaction time is 1-8 hours. After the reaction, remove the remaining solution and then wash with absolute ethanol. Carboxymethyl chitosan fiber is thus obtained after air drying.

Convert the air dried carboxymethyl chitosan fiber into non-woven fabric of 30-200g/m$^2$ after fiber opening, web formation and needling. Convert said non-woven fabric into anti-microbial carboxymethyl chitosan dressing by cutting, packaging and sterilizing.

Preferably, un-modified chitosan fiber is added after air dry but before fiber opening. The weight ratio of chitosan fiber to carboxymethyl chitosan fiber is 1:9 to 9:1. After mixing, strength of the non-woven wound dressing is improved.

More preferably, different additives are added into the wound dressing according to the present invention by different methods to change or improve the therapeutic property of the dressing. For example, nano-silver is added in the process of manufacturing said chitosan fiber to improve the anti-microbial property of the dressing. The production method is as follows:

In preparing chitosan fiber, add 0.1%-1% by weight of nano-silver particles into spinning solution.

Alternatively, the two-step method to produce carboxymethyl chitosan dressing can also be:

Chitosan fiber is made into non-woven fabric of 30-200g/m$^2$ after fiber opening, web formation and needling;

Contact said non-woven fabric with 40-50% NaOH solution with bath ratio being 1:20-60 for 0.5-6 hours at room temperature to obtain alkalized product, wash said alkalized product with absolute ethanol;

Contact said product with chloroacetic acid in isopropanol. The concentration of chloroacetic acid is 20-40%. Reaction temperature is 35-75°C. Reaction time is 1-8 hours. After the reaction, remove the remaining solution and then wash with absolute ethanol and air dry;

Carboxymethyl chitosan fabric is then made into carboxymethyl chitosan non-woven dressing by cutting, packaging and sterilizing.

Said dressing swells in water to become elastic gel material which is capable of absorbing about 30 times its own weight of water, which is much higher than other wound dressing.

Carboxymethyl chitosan fiber according to the present invention has a monofilament denier of 0.5-5dtex, a strength of 0.8-2.2cN/dtex. Degree of carboxymethyl substitution is between 0.4 and 0.8.

The therapeutic property of the wound dressing according to the present invention can also be improved by introducing nano-silver in the process of manufacturing carboxymethyl chitosan fiber or carboxymethyl chitosan non-woven fabric to improve the anti-microbial property of the dressing. The production method is as follows:

Contact said carboxymethyl chitosan fiber or carboxymethyl chitosan non-woven fabric obtained with silver nitrate in ethanol solvent to exchange sodium ion with silver ion. The concentration of silver nitrate solution is 0.5-10%. Reaction

temperature is 20-30°C. Reaction time is 0.5-2 hours. After the reaction, remove the remaining solution and then wash with absolute ethanol and air dry.

The hydrophilicity of the wound dressing according to the present invention can be improved by plasma treatment. The water-retaining property thereof can also be improved by crosslinking treatment.

The mechanism of plasma treatment in improving hydrophilic property of the wound dressing is opening the chemical bond on the surface of wound dressing to facilitate grafting hydrophilic group onto it. Plasma treatment is performed to greatly increase the hydrophilicity when inert gases or hydrophilic material are used as carrier. Plasma is used as an energy source to initiate polymerization. After a short period of irradiation from several seconds to several minutes at appropriate temperature, polymerization reaction is initiated in gas phase. Chain extention and termination is carried out in liquid and solid phase. The reaction initiated by low temperature plasma can significantly improve the property of medical biomaterials **characterized in that** (1) it is only effective on the surface of material upto to a depth of several dozens of nanometers and will not affect the property of basal materials; (2) it is capable of treating surfaces of different shapes; (3) it is highly germicidal and considered to be a satisfying surface treatment technique in the field of medical biomaterial.

In the plasma treatment of said wound dressing obtained under atmospheric pressure, the power of plasma discharge is 20W-100W, discharge time is 10 seconds-30 minutes. Suspend said dressing in certain hydrophilic solution for 5 seconds - 60 minutes.

In the plasma treatment of said wound dressing obtained at low temperature, the non-woven dressing prepared is juxtaposed in a plasma reactor which is connected to capacitive coupling.

In the hydrophilic grafting or polymerization of said dressing, modifying gas ventilated into vacuum reactor, modification pressure is 20-80Pa, modification time is 1-30 minutes;

Grafting polymerization is performed after modification, background vacuum is 2-8Pa, during glow discharge, the pressure of grafting polymerization is 10-60Pa, discharge time is 2-60 minutes and discharge power is 30-80W.

The dressing after plasma treatment swells in water to become elastic gel material which is capable of absorbing more than 30 times its own weight of water, which is higher than untreated wound dressing.

Crosslinking treatment of anti-microbial wound dressing overcomes a drawback of untreated wound dressing that a small portion of gel formed when contacting with liquid will dissolve after a period of time. The crosslinking treatments are as follows:

Heat crosslinking: place wound dressing of a certain size into vacuum oven, preheat for 1-5 hours at 50-80°C in vacuum; increase the temperature of the oven to 80-140°C, heat crosslinking for 2 hours-4 days. Keep vacuum and cool down the oven to room temperature, take out the dressing.

Chemical crosslinking: in the chemical crosslinking of the wound dressing prepared, place wound dressing of a certain size into glutaraldehyde solution, soak at room temperature for 5-60 minutes; alter the solution into acidic by acid and stay at room temperature for 2-48 hours, wash wound dressing thoroughly with phosphate buffer until un-crosslinked crosslinking agent is removed.

UV crosslinking: place wound dressing of a certain size under UV lights of 235-300nm wavelength; UV irradiation time for wound dressing is 5-60 minutes.

The practicality of wound dressings is significantly increased after plasma and crosslinking treatment as discussed above. The hydrophilicity of dressings is thus improved. The drawback of untreated wound dressing that a small portion of gel formed when contacting with liquid will dissolve after a period of time is also avoided. Said dressing can be applied to stop bleeding in surgical wounds, be left inside the body and absorbed afterwards. Said dressing can be applied in surgical wound, burn, and other chronic wound. Said dressing, when covering wound, is able to prevent water in body fluids from losing, provide a favorable moist environment for wound healing and maintain a fluid-free, maceration-free, germ-free wound surface. Said dressing is antiphlogistic, hemostatic and antalgic and facilitates wound healing.

The wound dressing according to the present invention is applicable in the field of surgical and burn treatment. Said wound dressing can be made into dressing for surgical wound, burn as well as chronic wound.

The wound dressing according to the present invention can be made into antiphlogistic and hemostatic wound wadding and drainage sliver which can be applied on surgical wounds to stop bleeding, be left inside human body and be absorbed afterwards.

The wound dressing according to the present invention can be used as an inner layer of composite dressings. When transparent or opaque film with adhesive on one side is used as an outer layer, said dressing can be made into island dressing for surgical wound.

Compared to prior arts, the present invention possesses the following advantages: the anti-microbial wound dressing according to the present invention can be applied in surgical wound, burn, and other chronic wound. Said dressing, when covering wound, is able to prevent water in body fluids from losing, provide a favorable moist environment of wound healing and maintain a fluid-free, maceration-free, germ-free wound surface. Said dressing is antiphlogistic, hemostatic and antalgic and facilitates wound healing.

In addition, the method according to the present invention solves the problem of hydrophobicity of carboxymethyl fiber when it is made into non-woven fabric by non-woven technique. Said method also solves the difficulty, in prior art, in fiber opening and web formation during consequent processing of non-woven fabric when carboxymethyl fiber is made into non-woven fabric by non-woven technique.

Embodiments

The present invention is further illustrated by the following examples:

Example 1:

Contact 100g chitosan fiber with 40% NaOH solution with bath ratio being 1:20 for 0.5 hour at room temperature to obtain alkalized chitosan fiber, wash said alkalized product with absolute ethanol; contact said product with chloroacetic acid in isopropanol, the concentration of chloroacetic acid is 20%, reaction temperature is 35°C, reaction time is 1 hour.

After the reaction, remove the remaining solution and then wash with absolute ethanol. Carboxymethyl chitosan fiber is obtained after air dry. Said product is made into non-woven fabric of 50g/m$^2$ after chopping, fiber opening, web formation and needling. Obtain dressing a1 by cutting, packaging and sterilizing said non-woven fabric. Dressing a1 is suitable for wound smaller than 10 cm$^2$.

Example 2:

Add chitosan fiber to carboxymethyl chitosan fiber which is used to manufacture a1. The weight ratio of chitosan fiber to carboxymethyl chitosan fiber is 9:1. The product is made into non-woven fabric of 100g/m$^2$ after mixing, chopping, fiber opening, web formation and needling. Obtain dressing a2 by cutting, packaging and sterilizing said non-woven fabric. Dressing a2 is suitable for relatively larger burn wound.

Example 3:

In making chitosan fiber for preparing a2, add 0.1% by weight of nano-silver particles into spinning solution and obtain non-woven wound dressing of blended carboxymethyl chitosan fiber and chitosan fiber containing nano-silver particles, which is labeled as a3.

Example 4:

Contact carboxymethyl chitosan fiber for preparing a1 with silver nitrate in ethanol solvent to exchange sodium ion with silver ion. The concentration of silver nitrate solution is 0.5%. Reaction temperature is 20°C. Reaction time is 0.5 hour. After the reaction, remove the remaining solution and then wash with absolute ethanol and air dry to obtain carboxymethyl chitosan fiber containing silver ion. The final product of non-woven wound dressing a4 is made of carboxymethyl chitosan fiber containing silver ion.

Example 5:

Contact 100g chitosan fiber with 45% NaOH solution with bath ratio being 1:40 for 1 hour at room temperature to obtain alkalized chitosan fiber; wash alkalized product with absolute ethanol; contact said product with chloroacetic acid in isopropanol, the concentration of chloroacetic acid is 30%, reaction temperature is 60°C, reaction time is 3 hours. After the reaction, remove the remaining solution and then wash with absolute ethanol. Obtain carboxymethyl chitosan fiber after air dry. The product is made into non-woven fabric of 50g/m$^2$ after chopping, fiber opening, web formation and needling. Obtain dressing b1 by cutting, packaging and sterilizing said non-woven fabric. Dressing b1 is suitable for wound smaller than 10 cm$^2$.

Example 6:

Add chitosan fiber to carboxymethyl chitosan fiber which is used to manufacture b1. The weight ratio of chitosan fiber to carboxymethyl chitosan fiber is 8:2. The product is made into non-woven fabric of 100g/m$^2$ after chopping, mixing, fiber opening, web formation and needling. Obtain dressing b2 by cutting, packaging and sterilizing said non-woven fabric. Dressing b2 is suitable for relatively larger burn wound.

Example 7:

In making chitosan fiber for preparing b2, add 0.5% by weight of nano-silver particles into spinning solution and obtain non-woven wound dressing of blended carboxymethyl chitosan fiber and chitosan fiber containing nano-silver particles, which is labeled as b3.

Example 8:

Contact carboxymethyl chitosan fiber for preparing b1 with silver nitrate in ethanol solvent to exchange sodium ion with silver ion. The concentration of silver nitrate solution is 5%. Reaction temperature is 25°C. Reaction time is 1 hour. After the reaction, remove the remaining solution and then wash with absolute ethanol and air dry to obtain carboxymethyl chitosan fiber containing silver ion. The final product of non-woven wound dressing b4 is made of carboxymethyl chitosan fiber containing silver ion.

Example 9:

Contact 100g chitosan fiber with 50% NaOH solution with bath ratio being 1:60 for 6 hours at room temperature to obtain alkalized chitosan fiber; wash said alkalized product with absolute ethanol; contact said product with chloroacetic acid in isopropanol, the concentration of chloroacetic acid is 40%, reaction temperature is 75°C, reaction time is 8 hours. After the reaction, remove the remaining solution and then wash with absolute ethanol. Obtain carboxymethyl chitosan fiber after air dry. The product is made into non-woven fabric of 50g/m$^2$ after chopping, fiber opening, web formation and needling. Obtain dressing c1 by cutting, packaging and sterilizing said non-woven fabric. Dressing c1 is suitable for wound smaller than 10 cm$^2$.

Example 10:

Add chitosan fiber to carboxymethyl chitosan fiber which is used to manufacture c1. The weight ratio of chitosan fiber to carboxymethyl chitosan fiber is 1:9. The product is made into non-woven fabric of 100g/m$^2$ after chopping, mixing, fiber opening, web formation and needling. Obtain dressing c2 by cutting, packaging and sterilizing said non-woven fabric. Dressing c2 is suitable for relatively larger burn wound.

Example 11:

In making chitosan fiber for preparing c2, add 1% by weight of nano-silver particles into spinning solution and obtain non-woven wound dressing of blended carboxymethyl chitosan fiber and chitosan fiber containing nano-silver particles, which is labeled as c3.

Example 12:

Contact carboxymethyl chitosan fiber for preparing c1 with silver nitrate in ethanol solvent to exchange sodium ion with silver ion. The concentration of silver nitrate solution is 10%. Reaction temperature is 30°C. Reaction time is 2 hours. After the reaction, remove the remaining solution and then wash with absolute ethanol and air dry to obtain carboxymethyl chitosan fiber containing silver ion. The final product of non-woven wound dressing c4 is made of carboxymethyl chitosan fiber containing silver ion.

Example 13:

Contact 100g chitosan non-woven fabric with 40% NaOH solution with bath ratio being 1:20 for 0.5 hour at room temperature to obtain alkalized non-woven chitosan fabric, wash alkalized product with absolute ethanol; contact said product with chloroacetic acid in isopropanol, the concentration of chloroacetic acid is 20%, reaction temperature is 35°C, reaction time is 1 hour. After the reaction, remove the remaining solution and then wash with absolute ethanol. Obtain carboxymethyl chitosan non-woven fabric after air dry. Obtain dressing d1 by cutting, packaging and sterilizing said non-woven fabric. Dressing d1 is suitable for wound smaller than 10 cm$^2$.

Example 14:

Contact carboxymethyl chitosan non-woven fabric for preparing d1 with silver nitrate in ethanol to exchange sodium ion with silver ion. The concentration of silver nitrate solution is 0.5%. Reaction temperature is 20°C. Reaction time is 0.5 hour. After the reaction, remove the remaining solution and then wash with absolute ethanol and air dry to obtain carboxymethyl chitosan non-woven fabric containing silver ion, labeled as d2.

Example 15:

Contact 100g chitosan non-woven fabric with 45% NaOH solution with bath ratio being 1:40 for 1 hour at room temperature to obtain alkalized non-woven chitosan fabric, wash alkalized product with absolute ethanol; contact said product with chloroacetic acid in isopropanol, the concentration of chloroacetic acid is 30%, reaction temperature is 60°C, reaction time is 3 hours. After the reaction, remove the remaining solution and then wash with absolute ethanol. Obtain carboxymethyl chitosan non-woven fabric after air dry. Obtain dressing e1 by cutting, packaging and sterilizing said non-woven fabric. Dressing e1 is suitable for wound smaller than 10 cm$^2$.

Example 16:

Contact carboxymethyl chitosan non-woven fabric for preparing e1 with silver nitrate in ethanol to exchange sodium ion with silver ion. The concentration of silver nitrate solution is 5%. Reaction temperature is 25°C. Reaction time is 1 hour. After the reaction, remove the remaining solution and then wash with absolute ethanol and air dry to obtain carboxymethyl chitosan non-woven fabric containing silver ion, labeled as e2.

Example 17:

Contact 100g chitosan non-woven fabric with 50% NaOH solution with bath ratio being 1:60 for 6 hours at room temperature to obtain alkalized non-woven chitosan fabric, wash said alkalized product with absolute ethanol; contact said product with chloroacetic acid in isopropanol, the concentration of chloroacetic acid is 40%, reaction temperature is 75°C, reaction time is 8 hours. After the reaction, remove the remaining solution and then wash with absolute ethanol. Obtain carboxymethyl chitosan non-woven fabric after air dry. Obtain dressing f1 by cutting, packaging and sterilizing said non-woven fabric. Dressing f1 is suitable for wound smaller than 10 cm$^2$.

Example 18:

Contact carboxymethyl chitosan non-woven fabric for preparing f1 with silver nitrate in ethanol to exchange sodium ion with silver ion. The concentration of silver nitrate solution is 10%. Reaction temperature is 30°C. Reaction time is 2 hours. After the reaction, remove the remaining solution and then wash with absolute ethanol and air dry to obtain carboxymethyl chitosan non-woven fabric containing silver ion, labeled as f2.

Example 19:

Juxtapose dressing a1 in a plasma reactor which is connected to capacitive coupling. In the hydrophilic grafting or polymerization of said dressing, oxygen is added into vacuum reactor, grafting polymerization is performed after modification, background vacuum is 2Pa, glow discharge. Modification pressure is 20Pa, modification time is 1 minute; the pressure of grafting polymerization is 10Pa, discharge time is 2 minutes and discharge power is 30W. The product obtained is A1.

Example 20:

Juxtapose dressing a1 in a plasma reactor which is connected to capacitive coupling. In the hydrophilic grafting or polymerization of said dressing, nitrogen or hydrogen is added into vacuum reactor, grafting polymerization is performed after modification, background vacuum is 6Pa, glow discharge. Modification pressure is 40Pa, modification time is 15 minutes; the pressure of grafting polymerization is 30Pa, discharge time is 20 minutes and discharge power is 50W. The product obtained is B1.

Example 21:

Juxtapose dressing a1 in a plasma reactor which is connected to capacitive coupling. In the hydrophilic grafting or polymerization of said dressing, inert gas is added into vacuum reactor, grafting polymerization is performed after modification, background vacuum is 8Pa, glow discharge. Modification pressure is 80Pa, modification time is 30 minutes; the pressure of grafting polymerization is 60Pa, discharge time is 60 minutes and discharge power is 80W. The product obtained is C1.

Example 22:

Place dressing a1 into vacuum oven for heat crosslinking, vacuum for 1 hour at 50°C; increase the temperature of the oven to 80°C, heat crosslinking for 2 hours; keep vacuum and cool down the oven to room temperature, take out the dressing. Sterilize the treated dressing and obtain product A2, keep it in storage.

Example 23:

Place dressing a1 into vacuum oven for heat crosslinking, vacuum for 3 hours at 60°C; increase the temperature of the oven to 120°C, heat crosslinking for 1 day; keep vacuum and cool down the oven to room temperature, take out the dressing. Sterilize the treated dressing and obtain product B2, keep it in storage.

Example 24:

Place dressing a1 into vacuum oven for heat crosslinking, vacuum for 5 hours at 80°C; increase the temperature of the oven to 140°C, heat crosslinking for 4 days; keep vacuum and cool down the oven to room temperature, take out the dressing. Sterilize the treated dressing and obtain product C2, keep it in storage.

Example 25:

Place dressing a1 into glutaraldehyde solution, soak it at room temperature for 5 minutes; alter the solution into acidic by acid and stay at room temperature for crosslinking for 2 hours, wash wound dressing thoroughly with phosphate buffer until un-crosslinked crosslinking agent is removed. Sterilize the treated dressing and obtain product A3, keep it in storage.

Example 26:

Place dressing a1 into glutaraldehyde solution, soak it at room temperature for 30 minutes; alter the solution into acidic by acid and stay at room temperature for crosslinking for 24 hours, wash wound dressing thoroughly with phosphate buffer until un-crosslinked crosslinking agent is removed. Sterilize the treated dressing and obtain product B3, keep it in storage.

Example 27:

Place dressing a1 into glutaraldehyde solution, soak it at room temperature for 60 minutes; alter the solution into acidic by acid and stay at room temperature for crosslinking for 48 hours, wash wound dressing thoroughly with phosphate buffer until un-crosslinked crosslinking agent is removed. Sterilize the treated dressing and obtain product C3, keep it in storage.

Example 28:

Perform UV crosslinking upon dressing a1: place dressing a1 under UV lights of 253.7nm wavelength; UV irradiation time for wound dressing is 5 minutes, sterilize the treated dressing and obtain product A4, keep it in storage.

Example 29:

Perform UV crosslinking upon dressing a1: place dressing a1 under UV lights of 253.7nm wavelength; UV irradiation time for wound dressing is 30 minutes, sterilize the treated dressing and obtain product B4, keep it in storage.

Example 30:

Perform UV crosslinking upon dressing a1: place dressing a1 under UV lights of 253.7nm wavelength; UV irradiation time for wound dressing is 60 minutes, sterilize the treated dressing and obtain product C4, keep it in storage.

Example 31:

Cut the non-woven dressing according to examples 1 to 10 into 10cm*10cm, obtain wound dressing for surgery, burn, and chronic wound after packaging and sterilization.

Example 32:

Cut the non-woven dressing according to examples 1 to 10 into 4cm*20cm, and stick it onto the adhesive side of a 9cm*25cm transparent film with medical adhesive on one side. Stick silicon paper onto the adhesive side. Obtain surgical island dressing after packaging and sterilization.

Example 33:

The non-woven fabric according to examples 1 to 10 can be made into antiphlogistic and hemostatic wound wadding after packaging and sterilization.

Example 34:

Cut the non-woven dressing according to examples 1 to 10 into 4cm*10cm, obtain antiphlogistic drainage sliver after packaging and sterilization.

The present invention is further illustrated by the following experiments:

Experiment 1: Anti-microbial test result 1 of carboxymethyl chitosan dressing

Test samples are products according to examples 1-18; control sample is normal medical gauze (Shanghai No. 21 fabric factory).

Test method is in accordance with AATCC100-1999, Evaluation of Anti-microbial Property of Fabrics.

Table 1, anti-microbial test result

| Group | E. coli (ATCC8099) | P. aeruginosa (ATCC27653) | Methicillin- resistant S. aureus | E. faecalis ( ATCC51575 ) |
|---|---|---|---|---|
| Control | 4.73% | 27.1% | 6.47% | 0 |
| a1 | >99% | >99% | >99% | >99% |
| a2 | >99% | >99% | >99% | >99% |
| a3 | >99% | >99% | >99% | >99% |
| a4 | >99% | >99% | >99% | >99% |
| b1 | >99% | >99% | >99% | >99% |
| b2 | >99% | >99% | >99% | >99% |
| b3 | >99% | >99% | >99% | >99% |
| b4 | >99% | >99% | >99% | >99% |
| c1 | >99% | >99% | >99% | >99% |
| c2 | >99% | >99% | >99% | >99% |
| c3 | >99% | >99% | >99% | >99% |
| c4 | >99% | >99% | >99% | >99% |
| d1 | >99% | >99% | >99% | >99% |
| d2 | >99% | >99% | >99% | >99% |
| e1 | >99% | >99% | >99% | >99% |
| e2 | >99% | >99% | >99% | >99% |
| f1 | >99% | >99% | >99% | >99% |
| f2 | >99% | >99% | >99% | >99% |
| Difference | mean >26% | | | |

Note 1. The difference between average bacterial colonies before and after vibration without sample being added is <10%. The test is credible.
2. Test sample is anti-microbial when the difference of anti-microbial rate between test sample and control is >26%.

According to the experiment result, the products according to examples 1-18 all have anti-microbial property.

Experiment 2: Liquid absorption test 1

Preparation of test samples: Obtain carboxymethyl chitosan non-woven fabric a1 according to Example 1 (test sample). Obtain low temperature plasma treated products A1, B1, C1 (test sample) according to Examples 19-21. Normal medical two-layer gauze is used as control (Shanghai No. 21 fabric factory). All the above samples are cut into 5cm*5cm.

Preparation of test solution: 8.298g sodium chloride and 0.368g calcium chloride are dissolved in 1000ml deionized water.

Test equipments

Analytical balance with an accuracy of 0.001g; incubator; Petri dish.

Test method

4.1 Get a sample with weight W1 in gram by balance;
4.2 Place the sample into a Petri dish, add test solution 40 times the weight of sample;
4.3 Place the Petri dish into 37°C incubator for 30 minutes;
4.4 Suspend the sample with forceps and stay in air for 30 seconds;
4.5 Obtain sample weight W2 in gram by balance;
4.6 Calculate liquid absorbency according to formula: $a = (W2-W1)/W1$, a is liquid absorbency
4.7 Calculate the arithmetic mean for two test results (see Table 2)

Table 2, liquid absorbency test

| Sample | Test 1 | | | Test 2 | | | a mean |
|---|---|---|---|---|---|---|---|
| | W1 | W2 | a | W1 | W2 | a | |
| a1 | 0.283 | 8.695 | 29.724 | 0.258 | 8.632 | 32.457 | 31.090 |
| A1 | 0.247 | 8.731 | 34.348 | 0.265 | 9.113 | 33.389 | 33.868 |
| B1 | 0.258 | 9.662 | 36.450 | 0.276 | 9.601 | 33.786 | 35.118 |
| C1 | 0.273 | 9.459 | 33.648 | 0.259 | 9.591 | 36.031 | 34.839 |
| Control | 0.155 | 2.514 | 15.219 | 0.158 | 2.594 | 15.418 | 15.318 |

Result: the liquid absorbency of a1 is 2.03 times that of the control sample, the liquid absorbency of A1 is 1.09 times that of a1 and 2.14 times that of the control sample, the liquid absorbency of B1 is 1.13 times that of a1 and 2.29 times that of the control sample, the liquid absorbency of C1 is 1.12 times that of a1 and 2.27 times that of the control sample. It proves that plasma treatment can increase hydrophilicity of wound dressings.

Experiment 3: wet breaking strength test

1. Preparation of test samples: Obtain crosslinking treated anti-microbial wound dressings according to Examples 22-30 (test samples). Obtain untreated anti-microbial wound dressing a1 according to Example 1 as control sample. All the above samples are cut into 2cm*4cm.
2. Place the sample into deionized water 40 times the weight of the sample in a Petri dish;
3. Place the Petri dish into 37°C incubator for 30 minutes;
4. Remove the Petri dish from incubator and suspend the sample with forceps and stay in air for 30 seconds;
5. Clip one end of the sample by the upper pliers of a universal testing machine type DXLL electronic tensile machine-20000. Hang the samples down to lower pliers and clip the pliers tightly. Samples should not be stretched or loosen at this time;
6. Pull the sample with tensile speed of 100mm/minute until sample is broken;
7. Record maximum breaking tenacity in the unit of N;
8. Calculate test result

```
wet breaking strength (N/cm) = maximum breaking tenacity

(N) / sample width (cm);
```

9. Display of test result
calculate the arithmetic mean for two test results (see Table 3)

Table 3 wet breaking strength test

| Sample | Test 1 | | Test 2 | | Mean N/cm |
|---|---|---|---|---|---|
| | N | N/cm | N | N/cm | |
| B2 | 4.38 | 2.190 | 4.35 | 2.175 | 2.183 |
| B3 | 4.49 | 2.245 | 4.53 | 2.265 | 2.255 |
| B4 | 4.51 | 2.255 | 4.53 | 2.265 | 2.260 |
| C2 | 6.49 | 3.245 | 6.53 | 3.265 | 3.255 |
| C3 | 6.42 | 3.210 | 6.49 | 3.245 | 3.228 |
| C4 | 6.49 | 3.245 | 6.51 | 3.255 | 3.250 |
| D2 | 1.34 | 0.670 | 1.37 | 0.685 | 0.678 |
| D3 | 1.40 | 0.700 | 1.39 | 0.695 | 0.698 |
| D4 | 1.31 | 0.655 | 1.38 | 0.690 | 0.672 |
| a1 | * | - | * | - | - |
| Note: 1. Gel formed when the control sample contacting with solution dissolves partially after a period of time, thus wet breaking strength is unavailable. 2. Gel formed when the test samples contacting with solution do not dissolve after a period time, thus wet breaking strength is available which is more than 3N/cm after chemical crosslinking. | | | | | |

## Claims

1. A method for the preparation of an anti-microbial wound dressing, comprising preparing carboxymethyl chitosan non-woven fabric from chitosan fiber which is then made into wound dressing by cutting, packaging and sterilizing, **characterized in that** the steps of preparing carboxymethyl chitosan non-woven fabric from chitosan fiber comprising:

   contacting chitosan fiber with 40-50% NaOH solution with bath ratio being 1:20-60 for 0.5-6 hours at room temperature to obtain alkalized chitosan fiber, washing said alkalized product with absolute ethanol;
   contacting said product with chloroacetic acid in isopropanol with the concentration of chloroacetic acid being 20-40%, reaction temperature being 35-75°C, reaction time being 1-8 hours;
   removing the remaining solution after the reaction, and then washing with absolute ethanol to obtain carboxymethyl chitosan fiber after air dry;
   preparing non-woven fabric by fiber chopping, opening, web formation and needling.

2. A method for the preparation of an anti-microbial wound dressing according to claim 1, **characterized in that**: contacting carboxymethyl chitosan fiber with silver nitrate in ethanol solvent to exchange sodium ion with silver ion with the concentration of silver nitrate solution being 0.5-10%, reaction temperature being 20-30°C, and reaction time being 0.5-2 hours. After the reaction, removing the remaining solution and then washing with absolute ethanol and air dry.

3. A method for the preparation of an anti-microbial wound dressing according to claims 1 or 2, **characterized in that**:

   adding chitosan fiber after air dry but before fiber opening, the weight ratio of chitosan fiber to carboxymethyl chitosan fiber being 1:9 to 9:1.

4. A method for the preparation of an anti-microbial wound dressing according to claim 3, **characterized in that**: In preparing chitosan fiber, adding 0.1%-1% by weight of nano-silver particles into spinning solution.

5. A method for the preparation of an anti-microbial wound dressing, comprising preparing carboxymethyl chitosan non-woven fabric from chitosan fiber which is then made into wound dressing by cutting, packaging and sterilizing, **characterized in that** the steps of preparing carboxymethyl chitosan non-woven fabric from chitosan fiber is as follows:

preparing non-woven fabric from chitosan fiber by fiber chopping, opening, web formation and needling, contacting said non-woven fabric with 40-50% NaOH solution with bath ratio being 1:20-60 for 0.5-6 hours at room temperature to obtain alkalized product, washing said alkalized product with absolute ethanol; contacting said product with chloroacetic acid in isopropanol with the concentration of chloroacetic acid being 20-40%, reaction temperature being 35-75°C, and reaction time being 1-8 hours; removing the remaining solution after the reaction, and then washing with absolute ethanol and air dry to obtain carboxymethyl chitosan non-woven fabric.

6. A method for the preparation of an anti-microbial wound dressing according to claim 5, **characterized in that**: contacting carboxymethyl chitosan fiber with silver nitrate in ethanol solvent to exchange sodium ion with silver ion with the concentration of silver nitrate solution being 0.5-10%, reaction temperature being 20-30°C, and reaction time being 0.5-2 hours. After the reaction, remove the remaining solution and then wash with absolute ethanol and air dry.

7. A method for the preparation of an anti-microbial wound dressing according to any one of claims 1-6, **characterized in that**:

performing low temperature plasma treatment on said wound dressing product, juxtaposing said wound dressing in a plasma reactor which is connected to capacitive coupling; hydrophilic grafting or polymerizing said dressing, adding modifying gas into vacuum reactor, background vacuum being 2-8Pa, glowing discharge, modification pressure being 20-80 Pa, modification time being 1-30 minutes; performing grafting polymerization after modification, background vacuum being 2-8Pa, during glow discharge, the pressure of grafting polymerization being 10-60Pa, discharge time being 2-60 minutes and discharge power being 30-80W.

8. A method for the preparation of an anti-microbial wound dressing according to any one of claims 1-6, **characterized in that**: performing heat crosslinking upon said wound dressing product, placing wound dressing of a certain size into vacuum oven, preheating for 1-5 hours at 50-80°C in vacuum; increasing the temperature of the oven to 80-140°C, heat crosslinking for 2 hours-4 days, keeping vacuum and cooling down the oven to room temperature, taking out the material.

9. A method for the preparation of an anti-microbial wound dressing according to any one of claims 1-6, **characterized in that**: performing chemical crosslinking upon said wound dressing product, placing wound dressing of a certain size into glutaraldehyde solution, soaking at room temperature for 5-60 minutes; adjusting the solution into acidic by acid and staying at room temperature for 2-48 hours, washing wound dressing thoroughly with phosphate buffer until un-crosslinked crosslinking agent being removed.

10. A method for the preparation of an anti-microbial wound dressing according to any one of claims 1-6, **characterized in that**: performing UV crosslinking upon said wound dressing product, placing wound dressing of a certain size under UV lights, UV irradiation time for wound dressing being 5-60 minutes.

11. Use of the anti-microbial wound dressing according to any one of claims 1 to 10 for producing surgical wound, burn, and other chronic wound dressing.

12. Use of the anti-microbial wound dressing according to any one of claims 1 to 10 for producing antiphlogistic and hemostatic wound wadding.

13. Use of the anti-microbial wound dressing according to any one of claims 1 to 10 for producing antiphlogistic drainage sliver.

14. Use of the anti-microbial wound dressing according to any one of claims 1 to 10 for producing surgical dressing as an inner layer of the composite dressing.

**Patentansprüche**

1. Verfahren zum Herstellen eines antimikrobiellen Wundverbands, umfassend das Herstellen von Carboxymethylchitosan-Vliesstoff aus Chitosanfasern, der anschließend durch Schneiden, Verpacken und Sterilisieren zu einem Wundverband verarbeitet wird, **dadurch gekennzeichnet, dass** die Schritte des Herstellens von Carboxymethylchitosan-Vliesstoff aus Chitosanfasern umfassen:

   Kontaktieren von Chitosanfasern mit einer 40-50%-igen NaOH-Lösung mit einem Flottenverhältnis von 1:20-60 für 0,5-6 Stunden bei Raumtemperatur, um alkalierte Chitosanfasern zu erhalten, Waschen des alkalierten Produkts mit absolutem Ethanol;
   Kontaktieren des Produkts mit Chloressigsäure in Isopropanol, wobei die Konzentration der Chloressigsäure 20-40 % beträgt, die Reaktionstemperatur 35-75 °C beträgt und die Reaktionszeit 1-8 Stunden beträgt;
   Entfernen der verbleibenden Lösung nach der Umsetzung und anschließend Waschen mit absolutem Ethanol, um nach Lufttrocknen Carboxymethylchitosanfasern zu erhalten;
   Herstellen von Vliesstoff durch Schneiden der Fasern, Auflockern, Bahnbildung und Vernadeln.

2. Verfahren zum Herstellen eines antimikrobiellen Wundverbands gemäß Anspruch 1, **gekennzeichnet durch**:

   Kontaktieren von Carboxymethylchitosanfasern mit Silbernitrat in Ethanol-Lösungsmittel, um Natriumionen gegen Silberionen auszutauschen, wobei die Konzentration der Silbernitratlösung 0,5-10 % beträgt, die Reaktionstemperatur 20-30 °C beträgt und die Reaktionszeit 0,5-2 Stunden beträgt. Nach der Umsetzung Entfernen der verbleibenden Lösung und anschließend Waschen mit absolutem Ethanol und Lufttrocknen.

3. Verfahren zum Herstellen eines antimikrobiellen Wundverbands gemäß Ansprüchen 1 oder 2, **gekennzeichnet durch**:

   Zugeben von Chitosanfasern nach dem Lufttrocknen aber vor dem Auflockern der Fasern, wobei das Gewichtsverhältnis von Chitosanfasern zu Carboxymethylchitosanfasern 1:9 bis 9:1 beträgt.

4. Verfahren zum Herstellen eines antimikrobiellen Wundverbands gemäß Anspruch 3, **gekennzeichnet durch**:

   bei der Herstellung der Chitosanfasern Zugeben von 0,1-1 Gew.-% Silber-Nanoteilchen zu der Spinnlösung.

5. Verfahren zum Herstellen eines antimikrobiellen Wundverbands, umfassend das Herstellen von Carboxymethylchitosan-Vliesstoff aus Chitosanfasern, der anschließend durch Schneiden, Verpacken und Sterilisieren zu einem Wundverband verarbeitet wird, **dadurch gekennzeichnet, dass** die Schritte des Herstellens von Carboxymethylchitosan-Vliesstoff aus Chitosanfasern folgendermaßen sind:

   Herstellen von Vliesstoff aus Chitosanfasern durch Schneiden der Fasern, Auflockern, Bahnbildung und Vernadeln;
   Kontaktieren des Vliesstoffs mit einer 40-50%-igen NaOH-Lösung mit einem Flottenverhältnis von 1:20-60 für 0,5-6 Stunden bei Raumtemperatur, um ein alkaliertes Produkt zu erhalten, Waschen des alkalierten Produkts mit absolutem Ethanol;
   Kontaktieren des Produkts mit Chloressigsäure in Isopropanol, wobei die Konzentration der Chloressigsäure 20-40 % beträgt, die Reaktionstemperatur 35-75 °C beträgt und die Reaktionszeit 1-8 Stunden beträgt;
   Entfernen der verbleibenden Lösung nach der Umsetzung und anschließend Waschen mit absolutem Ethanol und Lufttrocknen, um Carboxymethylchitosan-Vliesstoff zu erhalten.

6. Verfahren zum Herstellen eines antimikrobiellen Wundverbands gemäß Anspruch 5, **gekennzeichnet durch**:

   Kontaktieren von Carboxymethylchitosanfasern mit Silbernitrat in Ethanol-Lösungsmittel, um Natriumionen gegen Silberionen auszutauschen, wobei die Konzentration der Silbernitratlösung 0,5-10 % beträgt, die Reaktionstemperatur 20-30 °C beträgt und die Reaktionszeit 0,5-2 Stunden beträgt. Nach der Umsetzung Entfernen der verbleibenden Lösung und anschließend Waschen mit absolutem Ethanol und Lufttrocknen.

7. Verfahren zum Herstellen eines antimikrobiellen Wundverbands gemäß einem der Ansprüche 1-6, **gekennzeichnet durch**:

Durchführen einer Tieftemperatur-Plasmabehandlung des Wundverbandprodukts, nebeneinander Anordnen des Wundverbands in einem Plasmareaktor, der mit einer kapazitiven Kopplung verbunden ist;
hydrophiles Pfropfen oder Polymerisieren des Verbands, Zugeben von Modifizierungsgas in den Vakuumreaktor, wobei das Hintergrundvakuum 2-8 Pa beträgt, Glimmentladung, wobei der Modifizierungsdruck 20-80 Pa beträgt und die Modifizierungszeit 1-30 Minuten beträgt;
Durchführen einer Pfropfpolymerisation nach dem Modifizieren, wobei das Hintergrundvakuum 2-8 Pa beträgt, während einer Glimmentladung, wobei der Druck bei der Pfropfpolymerisation 10-60 Pa beträgt, die Entladungszeit 2-60 Minuten beträgt und die Entladungsleistung 30-80 W beträgt.

8. Verfahren zum Herstellen eines antimikrobiellen Wundverbands gemäß einem der Ansprüche 1-6, **gekennzeichnet durch**:

   Durchführen einer Wärmevernetzung an dem Wundverbandprodukt, Anordnen von Wundverband einer bestimmten Größe in einem Vakuumofen, 1-5 Stunden Vorwärmen bei 50-80 °C unter Vakuum;
   Erhöhen der Temperatur des Ofens auf 80-140 °C, 2 Stunden bis 4 Tage Wärmevernetzen, wobei das Vakuum gehalten wird, und Abkühlen des Ofens auf Raumtemperatur, Entnehmen des Materials.

9. Verfahren zum Herstellen eines antimikrobiellen Wundverbands gemäß einem der Ansprüche 1-6, **gekennzeichnet durch**:

   Durchführen einer chemischen Vernetzung an dem Wundverbandprodukt, Anordnen von Wundverband einer bestimmten Größe in einer Glutaraldehydlösung, 5-60 Minuten Tränken bei Raumtemperatur; saures Einstellen der Lösung **durch** Säure, 2-48 Stunden Halten bei Raumtemperatur, gründliches Waschen des Wundverbands mit Phosphatpuffer, bis nichtvernetztes Vernetzungsmittel entfernt ist.

10. Verfahren zum Herstellen eines antimikrobiellen Wundverbands gemäß einem der Ansprüche 1-6, **gekennzeichnet durch**:

    Durchführen einer UV-Vernetzung an dem Wundverbandprodukt, Anordnen von Wundverband einer bestimmten Größe unter UV-Lampen, wobei die UV-Bestrahlungszeit des Wundverbands 5-60 Minuten beträgt.

11. Verwendung des antimikrobiellen Wundverbands gemäß einem der Ansprüche 1 bis 10 zur Herstellung von chirurgischem Wund-, Verbrennungs- und anderem chronischen Wundverband.

12. Verwendung des antimikrobiellen Wundverbands gemäß einem der Ansprüche 1 bis 10 zur Herstellung von antiphlogistischer und hämostatischer Wundwatte.

13. Verwendung des antimikrobiellen Wundverbands gemäß einem der Ansprüche 1 bis 10 zur Herstellung von antiphlogistischem Drainageband.

14. Verwendung des antimikrobiellen Wundverbands gemäß einem der Ansprüche 1 bis 10 zur Herstellung von chirurgischem Verband als innere Schicht eines Kompositverbands.

**Revendications**

1. Procédé pour la préparation d'un pansement antimicrobien, comprenant la préparation d'un non-tissé de carboxyméthylchitosane à partir de fibres de chitosane qui est ensuite transformé en pansement par découpe, conditionnement et stérilisation, **caractérisé en ce que** les étapes de préparation du non-tissé de carboxyméthylchitosane à partir de fibres de chitosane comprennent:

   la mise en contact de fibres de chitosane avec une solution de NaOH à 40-50 %, le rapport de bain étant de 1 : 20-60, pendant 0,5-6 heures à température ambiante pour obtenir des fibres de chitosane alcalisées, et le lavage dudit produit alcalisé avec de l'éthanol absolu;
   la mise en contact dudit produit avec de l'acide chloracétique dans de l'isopropranol, la concentration d'acide chloracétique étant de 20-40 %, la température de réaction étant de 35-75 °C, et le temps de réaction étant de 1-8 heures;
   le retrait de la solution restante après la réaction, puis un lavage avec de l'éthanol absolu pour obtenir des fibres de carboxyméthylchitosane après séchage à l'air; et
la préparation d'un non-tissé par découpage des fibres, ouverture, formation d'un voile et aiguilletage.

2. Procédé pour la préparation d'un pansement antimicrobien selon la revendication 1, **caractérisé par**: la mise en contact de fibres de carboxyméthylchitosane avec du nitrate d'argent dans un solvant à base d'éthanol pour échanger les ions sodium avec les ions argent, la concentration de la solution de nitrate d'argent étant de 0,5-10 %, la température de réaction étant de 20-30 °C, et le temps de réaction étant de 0,5-2 heures, et après la réaction, le retrait de la solution restante puis un lavage avec de l'éthanol absolu et un séchage à l'air.

3. Procédé pour la préparation d'un pansement antimicrobien selon la revendication 1 ou 2, **caractérisé par**: l'addition de fibres de chitosane après le séchage à l'air mais avant l'ouverture des fibres, le rapport pondéral des fibres de chitosane aux fibres de carboxyméthylchitosane étant de 1 : 9 à 9 : 1.

4. Procédé pour la préparation d'un pansement antimicrobien selon la revendication 3, **caractérisé par**: lors de la préparation des fibres de chitosane, l'addition de 0,1-1 % en poids de nanoparticules d'argent dans la solution de filage.

5. Procédé pour la préparation d'un pansement antimicrobien, comprenant la préparation d'un non-tissé de carboxyméthylchitosane à partir de fibres de chitosane qui est ensuite transformé en pansement par découpe, conditionnement et stérilisation, **caractérisé en ce que** les étapes de préparation du non-tissé de carboxyméthylchitosane à partir de fibres de chitosane sont comme suit:

préparation d'un non-tissé à partir de fibres de chitosane par découpage des fibres, ouverture, formation d'un voile et aiguilletage;
mise en contact dudit non-tissé avec une solution de NaOH à 40-50 %, le rapport de bain étant de 1 : 20-60, pendant 0,5-6 heures à température ambiante pour obtenir un produit alcalisé, et lavage dudit produit alcalisé avec de l'éthanol absolu;
mise en contact dudit produit avec de l'acide chloracétique dans de l'isopropranol, la concentration d'acide chloracétique étant de 20-40 %, la température de réaction étant de 35-75 °C, et le temps de réaction étant de 1-8 heures ; et
retrait de la solution restante après la réaction, puis lavage avec de l'éthanol absolu et séchage à l'air pour obtenir un non-tissé de carboxyméthylchitosane.

6. Procédé pour la préparation d'un pansement antimicrobien selon la revendication 5, **caractérisé par**: la mise en contact de fibres de carboxyméthylchitosane avec du nitrate d'argent dans un solvant à base d'éthanol pour échanger les ions sodium avec les ions argent, la concentration de la solution de nitrate d'argent étant de 0,5-10 %, la température de réaction étant de 20-30 °C, et le temps de réaction étant de 0,5-2 heures, et après la réaction, le retrait de la solution restante puis un lavage avec de l'éthanol absolu et un séchage à l'air.

7. Procédé pour la préparation d'un pansement antimicrobien selon l'une quelconque des revendications 1 à 6, **caractérisé en ce que**:

on effectue un traitement par plasma à basse température sur ledit pansement, en juxtaposant ledit pansement dans un réacteur à plasma qui est relié à un couplage capacitif;
on effectue un greffage hydrophile ou une polymérisation dudit pansement, en ajoutant un gaz de modification dans un réacteur sous vide, le vide résiduel étant de 2-8 Pa, décharge lumineuse, la pression de modification étant de 20-80 Pa, et le temps de modification étant de 1-30 minutes ; et
on effectue une polymérisation par greffage après la modification, le vide résiduel étant de 2-8 Pa pendant la décharge lumineuse, la pression de polymérisation par greffage étant de 10-60 Pa, le temps de décharge étant de 2-60 minutes et la puissance de décharge étant de 30-80 W.

8. Procédé pour la préparation d'un pansement antimicrobien selon l'une quelconque des revendications 1 à 6, **caractérisé en ce que**: on effectue une réticulation thermique sur ledit pansement, en plaçant un pansement d'une certaine taille dans un four à vide, en préchauffant pendant 1-5 heures à 50-80 °C sous vide, en faisant monter la température du four à 80-140 °C, en réticulant à chaud pendant 2 heures - 4 jours, en maintenant le vide et en refroidissant le four à température ambiante, et en retirant le matériau.

9. Procédé pour la préparation d'un pansement antimicrobien selon l'une quelconque des revendications 1 à 6, **ca-**

**ractérisé en ce que**: on effectue une réticulation chimique sur ledit pansement, en plaçant un pansement d'une certaine taille dans une solution de glutaraldéhyde, en laissant tremper à température ambiante pendant 5-60 minutes, en ajustant l'acidité de la solution avec un acide en restant à température ambiante pendant 2-48 heures, et en lavant minutieusement le pansement avec un buffer phosphate jusqu'à ce que l'agent de réticulation non réticulé ait été éliminé.

10. Procédé pour la préparation d'un pansement antimicrobien selon l'une quelconque des revendications 1 à 6, **caractérisé en ce que**: on effectue une réticulation par UV sur ledit pansement, en plaçant un pansement d'une certaine taille sous un rayonnement UV, le temps d'irradiation UV du pansement étant de 5-60 minutes.

11. Utilisation du pansement antimicrobien selon l'une quelconque des revendications 1 à 10 pour la fabrication d'un pansement pour plaies chirurgicales, brûlures et autres lésions chroniques.

12. Utilisation du pansement antimicrobien selon l'une quelconque des revendications 1 à 10 pour la fabrication d'ouate antiphlogistique et hémostatique.

13. Utilisation du pansement antimicrobien selon l'une quelconque des revendications 1 à 10 pour la fabrication d'un ruban de drainage antiphlogistique.

14. Utilisation du pansement antimicrobien selon l'une quelconque des revendications 1 à 10 pour la fabrication d'un pansement chirurgical, comme couche interne du pansement composite.

EP 1 859 816 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9416746 A
- WO 9902093 A
- CN 92106598
- CN 03153650
- CN 20040015093
- US 20050058694 A

### Non-patent literature cited in the description

- **L Chen et al.** Studies on Carboxymethyl Chitosan's Structure and Anti-microbial Capabilities. *Journal of Wuhan University,* April 2000